# EUROPÄISCHE PATENTANMELDUNG

(11) **EP 2 246 350 A1**
(43) Veröffentlichungstag der Anmeldung: **03.11.2010**
(21) Anmeldenummer: 09157914.4
(22) Anmeldetag: 15.04.2009
(51) Int. Cl.: C07D 471/04, C09B 5/62, G02F 1/1335, G03F 7/00

(54) **Fluor-substituierte Perylene für Farbfilter in LCD**

(71) Anmelder: LANXESS Deutschland GmbH, 51369 Leverkusen (DE)
(72) Erfinder: Feldhues, Ulrich Dr., 51465 Bergisch Gladbach (DE); Linke, Frank, 51369 Kölln (DE); Michaelis, Stephan Dr., 51519 Odenthal (DE); Pfützenreuter, Dirk, 51399 Burscheid (DE)

(57) **Zusammenfassung**

Verwendung von Fluor-substituierten Perylenen in Farbfiltern für Flüssigkristallanzeigen.

## Beschreibung

Die Erfindung betrifft die Verwendung von Fluor-haltigen Perylenen in Farbfiltern für LCD (liquid crystal displays = Flüssigkristallanzeige) sowie Präparationen solcher Farbmittel und ihre Verwendung zur Herstellung von Farbfiltern, die Farbfilter selbst sowie noch neue Perylene.

Farbfilter finden heute vornehmlich Anwendung in Flüssigkristallanzeigen wie Flüssigkristallbildschirmen, Farbauflösungsgeräten und Sensoren. Ein bekanntes Beispiel sind die Flachbildschirme für Personalcomputer, Fernsehgeräte und Videokameras. Es gibt verschiedene Methoden zur Herstellung der Farbfilter, die sich sowohl in der Auftragung der Farben als auch der Erzeugung der Farbelementmuster der Basisfarben rot, grün und blau neben schwarz unterscheiden. Die Applikation bzw. Auftragung der Farben kann z. B. durch Einfärben einer Trägerschicht (z. B. Gelatine) mittels löslichen Farbstoffen oder Pigmenten ("Dye Method", "Dye Dispersion Method"), Siebdruck, Offsetdruck oder Tintenstrahldruck von Pigmentpasten, -präparationen oder -tinten, Elektrodeposition von Photolacken auf Basis von Farbstoffen oder Pigmenten sowie insbesondere mittels der Pigment-Dispersionsmethode, bei der Pigmente verwendet werden, die entweder in einem Polyimidharz ("nicht-photosensitive Polyimidmethode") oder in einem Photolack ("photosensitive Acrylmethode") dispergiert sind, erfolgen. Verbunden mit den genannten Verfahren kommt sowohl der drucktechnisch direkten Erzeugung der Farbelementmuster als auch der indirekten, photolithographischen Erzeugung Bedeutung zu, letztere insbesondere bei der o. g. Pigment-Dispersionsmethode. Die Technik der Pigment-Dispersionsmethode in Form der "nichtphotosensitiven Polyimidmethode" ist beispielsweise in JP-A-11-217514 (1998) offenbart.

Bei der Pigment-Dispersionsmethode nach dem Photolackverfahren liegen die farbgebenden Pigmente in einem UV-härtbaren Photolack fein verteilt (dispergiert) vor. Der Photolack besteht dabei neben dem Pigment im Allgemeinen aus den Komponenten Bindemittelharz, polymerisierbares Monomer, Photoinitiator sowie gegebenenfalls einem Lösungsmittel. Die Herstellung erfolgt z. B. derart, dass zunächst das Pigment in Form eines Konzentrates mit Lösungsmittel und gegebenenfalls Bindemittelharz feinteilig dispergiert wird und unmittelbar vor der Applikation zusammen mit dem Monomer und dem Photoinitiator sowie gegebenenfalls weiterer Komponenten eingestellt wird. Der pigmentierte Photolack wird auf das Substrat, z. B. Glas, gleichmäßig aufgetragen, z. B. mittels des sogenannten "spin-coating"-Verfahrens, vorgetrocknet, mittels der Photomaske mit UV-Licht belichtet, mittels einer in der Regel anorganisch alkalischen Lösung zu den gewünschten Farbelementmustern entwickelt, die Beschichtung gereinigt und gegebenenenfalls nachgehärtet. Dieser Prozess wird für jede Farbe wiederholt, in der Regel also dreimal für eine Trichromie z. B. in den Farben rot, grün und blau.

Die Vorteile bei der Verwendung von Pigmenten in Verbindung mit der Pigment-Dispersionsmethode liegen in der verbesserten Licht-, Feuchtigkeits- und Temperaturbeständigkeit der Farbfilter im Vergleich zu Farbstoff-basierten Beschichtungssystemen. Demgegenüber sind die Transparenz und Farbreinheit der Beschichtungen auf Basis von Pigmenten, unabhängig vom Beschichtungsverfahren, noch nicht zufriedenstellend. Insbesondere wenn verschiedene Pigmente in Mischung zur Nuancierung auf die gewünschten Farbortwerte im Photolack eingearbeitet werden, kommt es zu unerwünschten Brillanz- und Transparenzverlusten, so dass als Folge die Anzeigen bzw. Bildschirme (LCD) mit einem erhöhten Energieaufwand betrieben werden müssen.

Die Verwendung von Pigmenten vom Typ Perylentetracarbonsäurediimid wie C.I. Pigment Rot 149 bzw. Diketopyrrolopyrrol wie C.I. Pigment Rot 254 bzw. Anthrachinone wie C.I. Pigment Rot 177 in Farbfiltern ist prinzipiell bekannt. Die Mitverwendung von Perylenpigmenten wie C.I. Pigment Rot 149 ist beispielsweise zusammen mit anderen Pigmenten in Farbfiltern bereits in EP-A-1146087 erwähnt.

In EP 1004941 werden Mischkristalle von Chinacridonen wie C.I. Pigment Violett 19 und C.I. Pigment Rot 122 und ihre Verwendung u.a. für Colorfilter beschrieben. Neben dem einzigen konkreten Mischkristall werden allgemein auch solche mit durch Fluor am Chinacridonkern substituierte Chinacridone erwähnt.

In EP 1843407 werden durch Fluor am Perylenkern substituierte flüssig-kristalline Perylene als Halbleiter für Transistoren und Solarzellen offenbart.

In WO 2008/128934 werden spezielle Fluor-haltige Perylene und ihre Verwendung für EPD (elektrophoretische Displays) offenbart.

In JP 2002348493 werden Mischungen von ggf. durch Fluor am Chinacridonkern substituierte Chinacridone und ihrer sulfonierten Derivate für Colorfilter offenbart. Diese Pigmenttypen zeichnen sich teilweise bereits durch gute Lichtechtheit und Farbstärke aus. Transparenz und Farbreinheit sind noch nicht zufrieden stellend. Im übrigen weist die Herstellung von Mischkristallen (feste Lösungen) häufig Reproduzierbarkeits-Probleme hinsichtlich der Qualität auf, die sich dann insbesondere auf Transparenz und Farbreinheit, aber auch auf Farbstärke und Lichtechtheit nachteilig auswirken können.

Die Aufgabe der vorliegenden Erfindung ist demnach die Bereitstellung von roten organischen Pigmenten in Farbfiltern für LCD, die diese Nachteile nicht aufweisen.

Gegenstand der Erfindung ist demnach die Verwendung von Fluor-substituierten Perylenen in Farbfiltern für LCD. Äquivalent dazu ist ein Verfahren zum Pigmentieren von Farbfiltern für LCD, das dadurch gekennzeichnet ist, das der Farbfilter mit Fluor-substituierten Perylenen pigmentiert wird. Alle bevorzugten Ausführungsformen für die Verwendung sollen nachfolgend auch für das Verfahren zum Pigmentieren gelten. Bevorzugte Fluor-substituierte Perylene entsprechen der Formel (I), 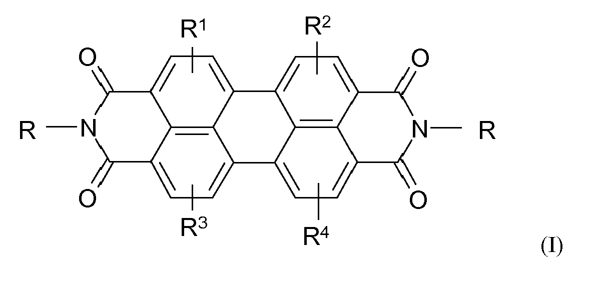 worin
- R: für H oder für einen ggf. substituierten organischen Rest steht,
- R¹, R², R³ und R⁴: unabhängig voneinander für H, F, Cl, Br oder für einen ggf. substituierten organischen Rest stehen und wenigstens einer der Reste R, R¹, R², R³ und R⁴ für Fluor steht oder wenigstens ein Fluoratom enthält.

Besonders bevorzugt sind Perylene der Formel (I), worin
- R: für einen durch ein oder mehrere Fluoratome substituierten organischen Rest steht und
- R¹, R², R³ und R⁴: die obige Bedeutung haben.

Unter R¹ - R⁴ wird vorzugsweise H verstanden.

Unter "organischem Rest" in der Bedeutung von R wird vorzugsweise C₁-C₈-Alkyl, insbesondere Methyl oder ggf. durch C₁-C₈-Alkyl, Phenoxy und/oder C₁-C₈-Alkoxy substituiertes C₆-C₁₀-Aryl verstanden, das jeweils durch ein oder mehrere Fluoratome substituiert ist.

Insbesondere sind als Perylene der Formel (I) solche bevorzugt, die der Formel (Ia) entsprechen, 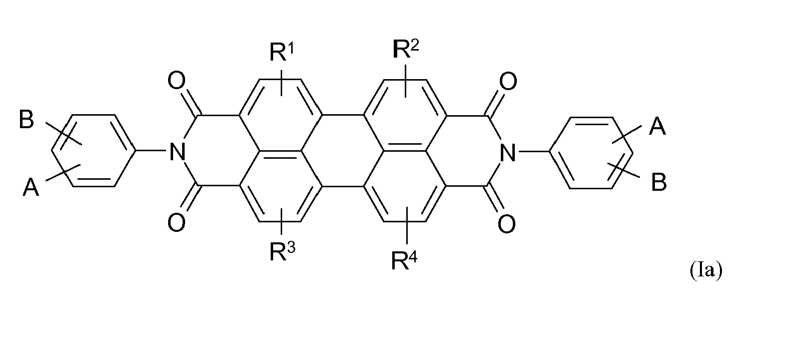 worin
- A: für Fluor oder einen durch ein oder mehrere Fluoratome substituierten organischen Rest steht,
- B: für H, F, Cl, Br oder für einen ggf. substituierten organischen Rest steht, der ggf. zusammen mit A einen Ring bilden kann, oder die Reste
- A und B: zusammen mit den C-Atomen des Phenylringes an denen sie gebunden sind einen annellierten Ring bilden und
- R¹, R², R³ und R⁴: die oben genannte Bedeutung haben.
Als bevorzugte durch Fluoratome substituierter organischer Rest in der Bedeutung von A und B ist C₁-C₈-Alkyl, C₁-C₈-Alkoxy, Phenyl oder Phenoxy zu nennen, das jeweils durch ein oder mehrere Fluoratome substituiert ist.

In der Formel (Ia) stehen R¹, R², R³, R⁴ vorzugsweise für Wasserstoff oder Fluor, der Rest B für H oder ggf. für Fluor-substituiertes C₁-C₈-Alkyl, insbesondere Methyl oder Trifluormethyl und A für Fluor oder für C₁-C₈-Alkyl, insbesondere Methyl, C₁-C₈-Alkoxy, insbesondere Methoxy, Phenyl oder Phenoxy, das vorzugsweise jeweils durch ein oder mehrere Fluoratome substituiert ist, oder

A und B gemeinsam ein Brückenglied der Formel -O-CF₂-O- , -O-CF₂-CF₂-O-, -O-CHF-CHF-O- oder -O-CF₂-CF₂-CF₂-O-.bedeuten.

Ebenfalls vorzugsweise bilden A und B gemeinsam eine durch ein oder mehrere Fluor-Atome substituierte Brücke, die mit zwei benachbarten C-Atomen des Benzolrings einen fünf-, sechs- oder siebengliedrigen Ring bildet, der carbocyclisch ist oder Heteroatome wie O, S oder N enthalten kann.

Geeignete durch Fluor substituierte C₁-C₈-Alkyl-Reste oder C₁-C₈-Alkoxy-Reste sind beispielsweise Methyl, Ethyl oder gegebenenfalls verzweigte Propyl-, Butyl-, Pentyl-, Hexyl- oder Octyl-Reste oder die entsprechenden Alkoxyreste, die mindestens ein Fluoratom tragen. Beispiele sind Fluormethyl, Trifluormethyl, 2-Fluorethyl, 2,2,2-Trifluorethyl, Pentafluorethyl, 2,2,3,3-Tetrafluorpropyl, Perfluorbutyl, Perfluoroctyl, insbesondere Perfluor-n-octyl und die entsprechenden Alkoxyreste.

Geeignete durch Fluor substituierte Phenyl- oder Phenoxyreste sind beispielsweise 2-, 3- oder 4-Fluorphenyl oder -phenoxy, 2,4- oder 3,4-Difluorphenyl oder -phenoxy, Pentafluorphenyl oder - phenoxy.

Geeignete ggf. substituierte organische Reste als mögliche Bedeutung von B sind vorzugsweise durch ein oder mehrere Fluoratome substituierte organische Reste, insbesondere solche, die unabhängig von A die gleiche Bedeutung wie der Rest A haben. Besonders bevorzugt sind die bevorzugten Bedeutungen von A, insbesondere Trifluormethyl.

Insbesondere bevorzugt kommen Pigmente der Formeln (II) bis (XII) verwendungsgemäß zum Einsatz,

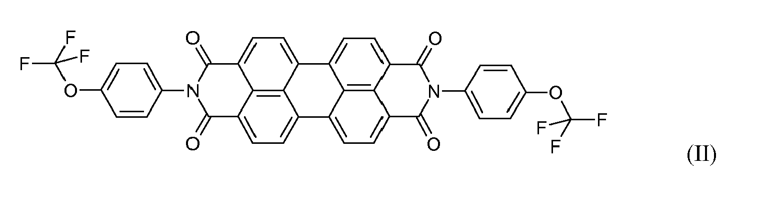

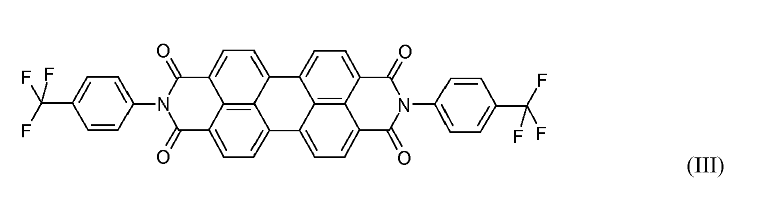

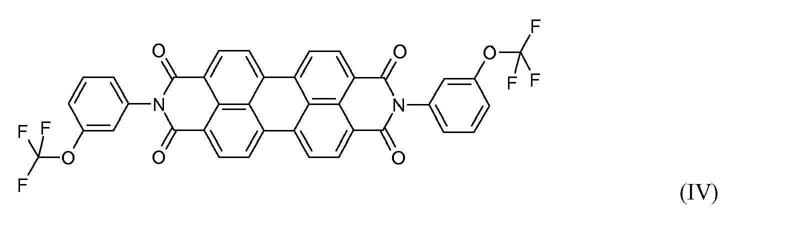

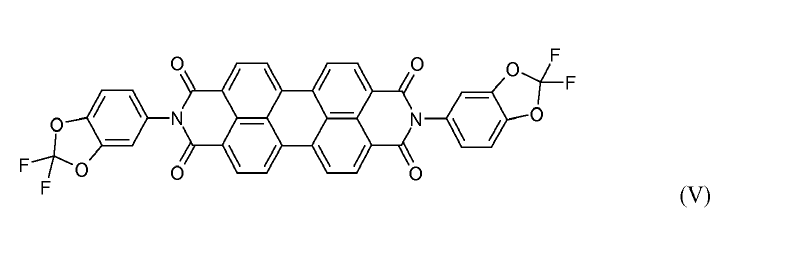

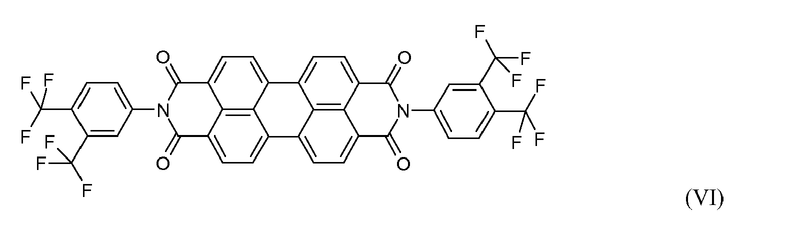

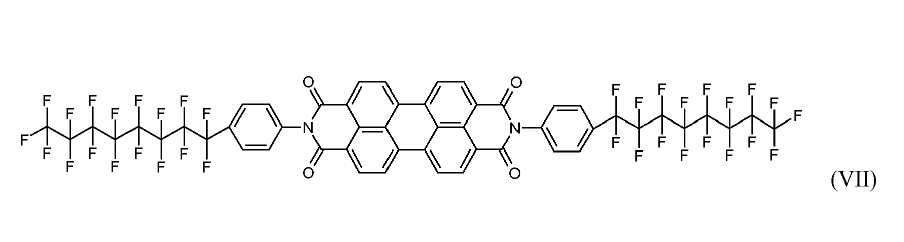

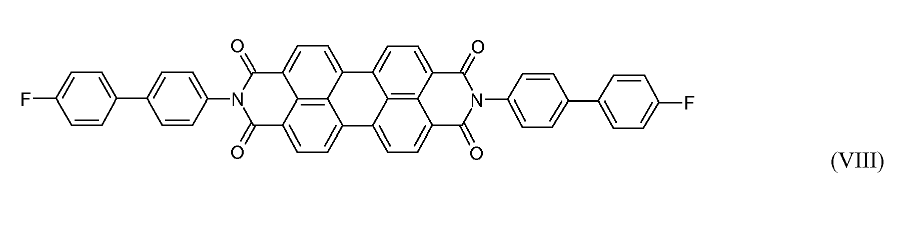

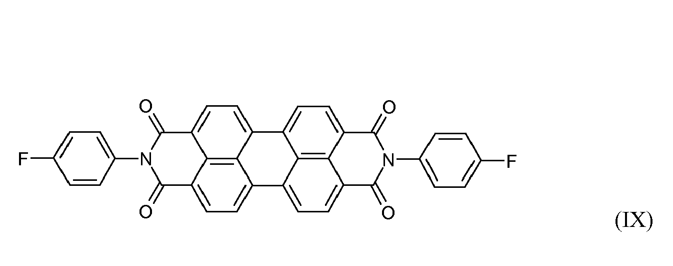

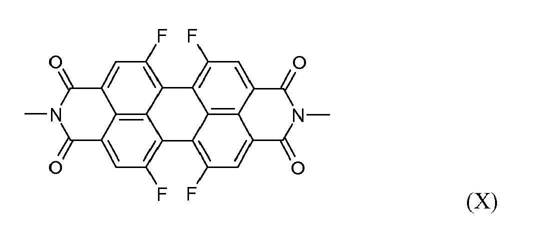

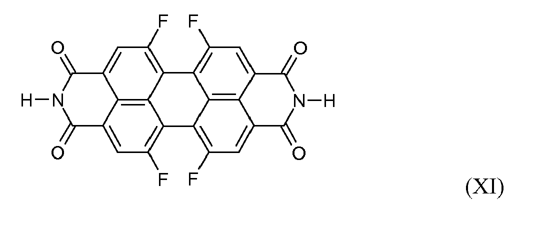

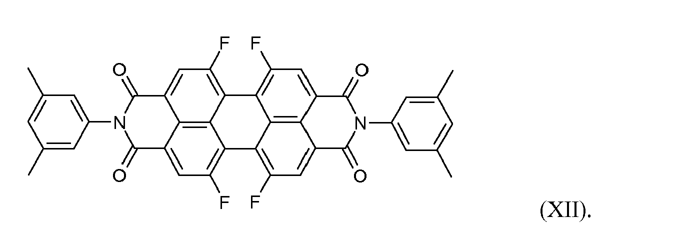

Die erfindungsgemäß verwendeten Pigmente besitzen vorzugsweise eine spezifische Oberfläche von 40 bis 200 m²/g, insbesondere von 60 bis 140 m²/g, ganz besonders bevorzugt von 70 bis 120 m²/g. Die Oberfläche wird nach DIN 66131 ermittelt: Bestimmung der spezifischen Oberfläche von Feststoffen durch Gasadsorption nach Brunauer, Emmett und Teller (B.E.T.)

Die erfindungsgemäß verwendeten Pigmente besitzen vorzugsweise eine Dispergierhärte von 10 bis 500, insbesondere von 20 bis 250 gemessen nach DIN 53775, Teil 7, wobei die Temperatur der Kaltwalzung 25°C und die Temperatur der Warmwalzung 150°C beträgt. Sämtliche in dieser Anmeldung angegebenen Dispergierhärten wurden nach dieser modifizierten DIN-Vorschrift bestimmt.

Die erfindungsgemäß verwendeten Pigmente besitzen vorzugsweise eine Partikelgröße von (Längsachse im Transmissions-Elektronen-Mikroskop) von 10 bis 200 nm, insbesondere von 20 bis 100 nm. Bevorzugt besitzen die erfindungsgemäß verwendeten Pigmente eine enge Partikelgrößenverteilung mit einer relativen Standardabweichung (Standardabweichung/Partikelgröße) < 50%, insbesondere < 35%, besonders bevorzugt < 20%. Bevorzugt besitzen die erfindungsgemäß verwendeten Pigmente ein Längen- zu Breitenverhältnis von 5 : 1 bis 1 : 1, insbesondere von 3 : 1 bis 1 : 1, besonders bevorzugt von 2 : 1 bis 1,2 : 1.

Die Partikelgröße und Oberfläche der erfindungsgemäß verwendeten Pigmente kann eingestellt werden durch Methoden, die an sich bekannt sind und beispielsweise in US 6,068,695 aufgeführt sind, wie z.B. Salzknetung oder Kugelmahlung und/oder ggf. nachgelagerte Finishingschritte wie z.B. Temperungen in wässrigen, organischen oder wässrig/organischen Lösungsmitteln mit oder ohne Additivzusatz.

Die erfindungsgemäß verwendeten Perylene Pigmente insbesondere die der Formel (I) können auch in Kombination mit anderen Pigmenten eingesetzt werden, beispielsweise zur Optimierung der optischen Eigenschaften der Farbfilter. Hinsichtlich der Auswahl anderer gegebenenfalls mitzuverwendender Pigmente besteht erfindungsgemäß keine Einschränkung. Es kommen sowohl anorganische als auch organische Pigmente in Frage.

Bevorzugte organische Pigmente sind z.B. solche der Monoazo-, Disazo-, verlackte Azo-, β-Naphthol-, Napthol AS-, Benzimidazolon-, Chinacridon-, Disazokondensations-, Azometallkomplex-, Isoindolin- und Isoindolinon-Reihe, ferner polycyclische Pigmente wie z.B. aus der Phthalocyanin-, Chinacridon-, Perylen-, Perinon-, Thioindigo-, Anthrachinon-, Dioxazin-, Chinophthalon- und Diketopyrrolopyrrol-Reihe. Außerdem verlackte Farbstoffe wie Ca-, Mg- und Al-Lacke von sulfonsäure- oder carbonsäuregruppenhaltigen Farbstoffen. Ganz besonders bevorzugt wird der mit Melamin interkallierte Nickelkomplex der Azobarbitursäure als mitzuverwendendes Pigment beansprucht.

Beispiele für gegebenenfalls mitzuverwendende andere organische Pigmente sind:
Color Index Pigment Yellow 12, 13, 14, 17, 20, 24, 74, 83, 86, 93, 94, 109, 110, 117, 125, 137, 138, 139, 147, 148, 150, 153, 154, 166, 173, 185,
Color Index Pigment Orange 13, 31, 36, 38, 40, 42, 43, 51, 55, 59, 61, 64, 65, 71, 72, 73,
Color Index Pigment Red 9, 97, 122, 123, 144, 149, 166, 168, 177, 179, 180, 192, 215, 216, 224, 254, 272,
Color Index Pigment Green 7, 10, 36, 37, 45, 58
Color Index Pigment Blue 15, 15:1, 15:2, 15:3, 15:4, 15:6, 16,
Color Index Pigment Violett 19, 23.

Als weitere nicht aus dem Color Index bekannte Pigmente sind beispielsweise das aus DE102005033581, insbesondere Beispiel 2, bekannte Melamin interkallierte Nickelazobarbitursäurekomplex Pigment der Formel 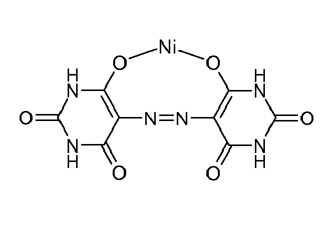 sowie seine tautomeren Formen zu nennen.

Ebenfalls sind sulfonierte Derivate der erfindungsgemäß verwendeten Pigmente, insbesondere solche der Formel (I) zu nennen.

Sofern "andere Pigmente" als die Fluor-substituierten Perylene, insbesondere solche der Formel (I) zusätzlich mitverwendet werden, beträgt der Anteil von "Pigment" im obigen Sinne entsprechend der Formel (I) vorzugsweise 1-99 Gew.-%, insbesondere 20-80 Gew.-%, bezogen auf die eingesetzte Gesamtmenge aller Pigmente.

Vorzugsweise mitzuverwendende Pigmente sind Pigment Rot 122, Pigment Rot 149, Pigment Rot 177, Pigment Rot 179, Pigment Rot 254, Pigment Violett 19, Pigment Gelb 138, Pigment Gelb 139, Pigment Gelb 150 oder ein mit Melamin interkalliertes Nickelazobarbitursäurekomplex-Pigment.

Besonders bevorzugt ist die Verwendung von Pigment-Mischungen, enthaltend neben wenigstens einem Fluor-substituierten Perylen, insbesondere wenigstens einem Pigment der Formel (I), wenigstens ein weiteres Rotpigment aus der Gruppe C.I. Pigment Rot 122, C.I. Pigment Rot 149, C.I. Pigment Rot 177, C.I. Pigment Rot 179, C.I. Pigment Rot 254 und C.I. Pigment Violett 19 in Farbfiltern für LCD.

Diese bevorzugt einzusetzende Mischung enthält ggf. zusätzlich ein oder mehrere Gelbpigmente oder Organgepigmente.

Die mitverwendeten Gelbpigmente bzw. Orangepigmente besitzen vorzugsweise eine Absorptionsbande im Bereich von 400 bis 520 nm.

Besonders enthält diese bevorzugte Mischung Gelbpigmente ausgewählt aus der Gruppe C.I. Pigment Gelb 138, C.I. Pigment Gelb 139, C.I. Pigment Gelb 150 und ein Melamin interkaliertes Nickelazobarbitursäurekomplex-Pigment.

Die Mischungen als solche sind ebenfalls Gegenstand dieser Erfindung.

Bevorzugte Mischungen enthalten neben wenigstens einem Fluor-substituierten Perylen, vorzugsweise solchen der Formel (I), insbesondere neben einem Pigment der Formel (Ia) wenigstens ein weiteres Rotpigment aus der Gruppe C.I. Pigment Rot 122, C.I. Pigment Rot 149, C.I. Pigment Rot 177, C.I. Pigment Rot 179, C.I. Pigment Rot 254 und C.I. Pigment Violett 19.

Sofern als "andere Pigmente" gelbe oder orange Pigmente eingesetzt werden, beträgt der Anteil dieser gelben oder orangen "anderen Pigmente" vorzugsweise 1 bis 50 Gew.-%, insbesondere 5 - 30 Gew.-%, bezogen auf die eingesetzte Gesamtmenge aller Pigmente.

Sofern als "andere Pigmente" rote Pigmente eingesetzt werden, beträgt der Anteil dieser roten "anderen Pigmente" vorzugsweise 1 bis 99 Gew.-%, insbesondere 20 - 80 Gew.-%, bezogen auf die eingesetzte Gesamtmenge aller Pigmente.

Die mit den erfindungsgemäßen Pigmenten oder ihren Mischungen hergestellten Farbfilter zeichnen sich insbesondere durch hohe Farbreinheit und excellente Transparenz aus.

Ebenfalls bevorzugt werden die Fluor-substituierten Perylene, insbesondere solche der Formel (I), in Form einer Mischung verwendet, die zusätzlich noch wenigstens ein Gelbpigment enthält. Vorzugsweise solche Gelbpigmente, die eine Absorptionsbande im Bereich von 400 bis 520 nm besitzen. Besonders enthält diese bevorzugte Mischung Gelbpigmente ausgewählt aus der Gruppe C.I. Pigment Gelb 138, C.I. Pigment Gelb 139, C.I. Pigment Gelb 150 und ein Melamin interkaliertes Nickelazobarbitursäurekomplex-Pigment. Auch diese Mischungen als solche sind ebenfalls Gegenstand dieser Erfindung.

Die Erfindung betrifft auch noch neue Perylene der Formel (II), (IV) und (V), wobei das der Formel (II) besonders bevorzugt ist.

Die erfindungsgemäße Verwendung der oben beschriebenen Pigmente bzw. Pigmentmischungen zur Herstellung von Farbfiltern für Flüssigkristallanzeigen sei im Folgenden am Beispiel der Pigment-Dispersionsmethode nach dem Photolackverfahren beschrieben.

Die erfindungsgemäße Verwendung der Fluor-substituierten Perylene, insbesondere auch der erfindungsgemäßen Pigmente zur Herstellung von Farbfiltern ist beispielsweise **dadurch gekennzeichnet, dass** das Pigment, gegebenenfalls mit einem Bindemittelharz und einem organischen Lösungsmittel, gegebenenfalls unter Zusatz eines Dispergiermittels, homogenisiert und anschließend kontinuierlich oder diskontinuierlich auf eine Teilchengröße nach Anzahl (elektronenmikroskopische Bestimmung) von 99,5 % <1000 nm, vorzugsweise 95 % <500 nm und insbesondere 90 % <200 nm nasszerkleinert wird. Als Nasszerkleinerungsverfahren kommen beispielsweise Rührer- oder Dissolverdispergierung, Mahlen mittels Rührwerkskugel- oder -perlmühlen, Kneter, Walzenstuhl, Hochdruckhomogenisierung oder Ultraschalldispergierung in Frage.

Während der Dispergierbehandlung oder im Anschluss daran erfolgt die Zugabe von mindestens einem photohärtbaren Monomeren und einem Photoinitiator. Im Anschluss an die Dispergierung kann noch weiteres Bindemittelharz, Lösungsmittel oder für Photolacke übliche Zuschlagstoffe eingebracht werden, wie es für die gewünschte photosensitive Beschichtungsmitteleinstellung (Photolack) zur Herstellung der Farbfilter erforderlich ist. Im Rahmen dieser Erfindung wird unter Photolack eine Präparation verstanden, die wenigstens ein photohärtbares Monomer und einen Photoinitiator zusätzlich zum Fluor-substituierten Perylen, insbesondere zum Perylen der Formel (I) enthält.

Als mögliche Dispergiermittel kommen allgemein handelsübliche wie beispielsweise polymere, ionogene oder nicht-ionogene Dispergiermittel bspw. auf Basis von Polycarbonsäuren oder Polysulfonsäuren, sowie Polyethylenoxid-Polypropylenoxid-Block-Copolymere in Betracht. Ferner können auch Derivate organischer Farbstoffe als Dispergiermittel oder Co-Dispergiermittel verwendet werden.

Bei der Herstellung der Farbfilter fallen daher zunächst "Zubereitungen" an, die bezogen auf die Zubereitung enthalten:
- wenigstens ein Fluor-substituiertes Perylen, insbesondere ein Perylen der Formel (I), welches im Sinne dieser Anmeldung als erfindungsgemäßes Pigment bezeichnet wird,
- gegebenenfalls ein oder mehrere andere Pigmente,
- gegebenenfalls ein Bindemittelharz,
- wenigstens ein organisches Lösungsmittel sowie
- gegebenenfalls ein Dispergiermittel.

In einer bevorzugten Ausführungsform enthält die Zubereitung (Angaben bezogen auf Zubereitung):

| | |
|---|---|
| 1 - 50 Gew.-% | wenigstens ein Fluor-substituiertes Perylen, insbesondere ein Perylen der Formel (I) |
| 0 - 50 Gew.-% | eine oder mehrerer anderer Pigmente |
| 0 - 20 Gew.-% | Bindemittelharz |
| 0 - 20 Gew.-% | Dispergiermittel |
| 10 - 94 Gew.-% | organisches Lösungsmittel. |

Die Beschichtung des Photolackes auf eine Platte zur Erzeugung der gefärbten Bildelementmuster kann entweder durch direkten oder indirekten Auftrag geschehen. Als Auftragsmethoden seien bspw. genannt: Ink-Jet, Roller-Coating, Spin-Coating, Spray-Coating, Dip-Coating und Air-Knife-Coating.

Als Platten kommen je nach Verwendung beispielsweise in Frage: transparente Gläser wie weiße oder blaue Glasplatte, mit Silikat beschichtete blaue Glasplatte, Kunstharzplatte oder -filme auf Basis von z.B. Polyester-, Polycarbonat-, Acryl-, oder Vinylchloridharz, ferner Metallplatten auf Basis von Aluminium, Kupfer, Nickel, oder Stahl sowie Keramikplatten oder Halbleiterplatten mit aufgebrachten photoelektrischen Transferelementen.

Die Auftragung erfolgt im Allgemeinen so, dass die Schichtdicke der erhaltenen photosensitiven Schicht bei 0,1 bis 10 µm liegt.

Im Anschluss an den Auftrag kann eine thermische Trocknung der Schicht erfolgen.

Die Belichtung erfolgt vorzugsweise, indem die photosensitive Schicht einem aktiven Lichtstrahl vorzugsweise in Form eines Bildmusters mittels Photomaske ausgesetzt wird. Hierdurch wird an den belichteten Stellen die Schicht gehärtet. Geeignete Lichtquellen sind z.B.: Hochdruck- und Ultrahochdruckquecksilberdampflampe, Xenon-, Metallhalogenid-, Fluoreszenzlampe sowie Laserstrahl im sichtbaren Bereich.

Durch die Entwicklung im Anschluss an die Belichtung wird der unbelichtete Teil der Beschichtung entfernt und man erhält die gewünschte Bildmusterform der Farbelemente. Übliche Entwicklungsmethoden umfassen das Besprühen mit oder Tauchen in wässrige alkalische Entwicklerlösung oder in ein organisches Lösungsmittel, das anorganische Alkali wie z.B. Natrium- oder Kaliumhydroxid, Natriummetasilikat oder organische Basen wie Monoethanolamin, Diethanolamin, Triethanolamin, Triethylamin oder deren Salze enthält.

Nach der Entwicklung erfolgt in der Regel eine thermische Nachtrocknung/-härtung der Bildmuster.

Als Bindemittelharze, die zusammen mit dem "Pigment" oder darauf basierender Pigmentpräparationen (also enthaltend Bindemittelharz und Pigment der Formel (I)) in Farbfiltern bzw. in den Zubereitungen zur Herstellung von Farbfiltern z.B. nach der Pigment-Dispersionsmethode, eingesetzt werden können, besteht erfindungsgemäß keine besondere Einschränkung, insbesondere kommen für die Anwendung in Farbfiltern an sich bekannte filmbildende Harze in Frage.

Beispielsweise kommen Bindemittelharze aus der Gruppe der Celluloseharze wie Carboxymethylhydroxyethylcellulose und Hydroxyethylcellulose, Acrylharze, Alkydharze, Melaminharze, Epoxidharze, Polyvinylalkohole, Polyvinylpyrrolidone, Polyamide, Polyamidimine und Polyimide in Frage.

Als Bindemittelharze kommen auch solche in Frage, die photopolymerisierbare, ungesättigte Bindungen enthalten. Die Bindemittelharze können beispielsweise solche aus der Gruppe der Acrylharze aufgebaut sein. Dabei sind insbesondere Mono- und Copolymere polymerisierbarer Monomere zu nennen wie z.B. (Meth)acrylsäuremethylester, (Meth)acrylsäureethylester, (Meth)acrylsäurepropylester, (Meth)acrylsäurebutylester, Styrol und Styrolderivate, ferner Copolymere zwischen carboxylgruppentragenden polymerisierbaren Monomeren wie (Meth)acrylsäure, Itaconsäure, Maleinsäure, Maleinsäureanhydrid, Maleinsäuremonoalkylester, insbesondere mit Alkyl von 1 bis 12 C-Atomen, und polymerisierbare Monomere wie (Meth)acrylsäure, Styrol und Styrolderivate, wie z.B. α-Methylstyrol, m- oder p-Methoxystyrol, p-Hydroxystyrol. Als Beispiele seien genannt Umsetzungsprodukte von carboxylgruppenhaltigen polymeren Verbindungen mit Verbindungen, die jeweils einen Oxiranring und eine ethylenisch ungesättigte Verbindung enthalten wie z.B. Glycidyl(meth)acrylat, Acrylglycidylether und Itaconsäuremonoalkylglycidylether usw., ferner Umsetzungsprodukte von carboxylgruppenhaltigen polymeren Verbindungen mit Verbindungen, die jeweils eine Hydroxylgruppe und eine ethylenisch ungesättigte Verbindung (ungesättigte Alkohole) enthalten wie Allylalkohol, 2-Buten-4-ol, Oleylalkohol, 2-Hydroxyethyl(meth)acrylat, N-Methylolacrylamid usw.; weiterhin können derartige Bindemittelharze auch ungesättigte Verbindungen, die freie Isocyanatgruppen besitzen, enthalten.

Im Allgemeinen liegt die Äquivalenz der Ungesättigtheit (Molgewicht Bindemittelharz pro ungesättigte Verbindung) der genannten Bindemittelharze bei 200 bis 3 000, insbesondere 230 bis 1 000, um sowohl eine ausreichende Photopolymerisierbarkeit und Härte des Films zu erreichen. Der Säurewert liegt im Allgemeinen bei 20 bis 300, insbesondere 40 bis 200, um eine genügende Alkali-Entwicklungsfähigkeit nach der Belichtung des Films zu erzielen.

Das mittlere Molgewicht der einzusetzenden Bindemittelharze liegt zwischen 1 500 und 200 000, insbesondere 10 000 bis 50 000 g/mol.

Die bei der erfindungsgemäßen Verwendung der Zubereitung für Farbfilter eingesetzten organischen Lösungsmittel sind z.B. Ketone, Alkylenglykolether, Alkohole und aromatische Verbindungen. Beispiele aus der Gruppe der Ketone sind: Aceton, Methylethylketon, Cyclohexanon etc.; aus der Gruppe der Alkylenglykol-ether: Methylcellosolve (Ethylenglykolmonomethylester), Butylcellosolve (Etylen-glykolmonobutylether) Methylcellosolveacetat, Ethylcellosolveacetat, Butyl-cellosolveacetat, Ethylenglykolmonopropylether, Ethylenglykolmonohexylether, Ethylenglykoldimethylether, Diethylenglykolethylether, Diethylenglykoldiethylether, Propylenglykolmonomethylether, Propylenglykolmonoethylether, Propylenglykol-monopropylether, Propylenglykolmonobutylether, Propylenglykolmonomethyl-etheracetat, Diethylenglykolmethyletheracetat, Diethylenglykolethyletheracetat, Diethylenglykolpropyletheracetat, Diethylenglykolisopropyletheracetat, Diethylen-glykolbutyletheracetat, Diethylenglykol-t-butyletheracetat, Triethylenglykolmethyl-etheracetat, Triethylenglykolethyletheracetat, Triethylenglykolpropyletheracetat, Triethylenglykolisopropyletheracetat, Triethylenglykolbutyletheracetat, Triethylen-glykol-t-butyletheracetat, etc.; aus der Gruppe der Alkohole: Methylalkohol, Ethylalkohol, Isopropylalkohol, n-Butylalkohol, 3-Methyl-3-methoxybutanol, etc.; aus der Gruppe der aromatischen Lösungsmittel Benzol, Toluol, Xylol, N-Methyl-2-Pyrrolidon, N-Hydroxymethyl-2-Essigsäureethylester, etc.

Weitere andere Lösungsmittel sind 1,2-Propandioldiacetat, 3-Methyl-3-methoxybutylacetat, Essigsäureethylester, Tetrahydrofuran, etc. Die Lösungsmittel können einzeln oder in Gemischen untereinander eingesetzt werden.

Die Erfindung betrifft weiterhin einen Photolack, enthaltend wenigstens ein Pigment im obigen Sinne oder wenigstens eine erfindungsgemäße Pigmentpräparation und wenigstens ein photohärtbares Monomer sowie wenigstens einen Photoinitiator.

Die photohärtbaren Monomere enthalten im Molekül wenigstens eine reaktive Doppelbindung und gegebenenfalls andere reaktive Gruppen.

Als photohärtbare Monomere seien in diesem Zusammenhang insbesondere reaktive Lösungsmittel bzw. sog. Reaktivverdünner verstanden z.B. aus der Gruppe der mono-, di-, tri- und multifunktionelle Acrylate und Methacrylate, Vinylether, sowie Glycidylether. Als zusätzlich enthaltene reaktive Gruppen kommen in Frage Allyl-, Hydroxy-, Phosphat-, Urethan-, sek. Amin- und N-Alkoxymethylgruppen. Derartige Monomere sind dem Fachmann bekannt und beispielsweise in *[*Römpp Lexikon, Lacke und Druckfarben, Dr. Ulrich Zorll, Thieme Verlag Stuttgart-New York, 1998, S. 491/492*]* oder unter dem Stichwort 'Reaktivverdünner' aufgeführt.

Die Auswahl der Monomere richtet sich insbesondere nach der Art und Intensität der verwendeten Strahlenart der Belichtung, der gewünschten Reaktion mit dem Photoinitiator und den Filmeigenschaften. Es können auch Kombinationen von Monomeren eingesetzt werden.

Als Photoreaktionsstarter oder Photoinitiatoren seien Verbindungen verstanden, die infolge der Adsorption sichtbarer oder ultravioletter Strahlung reaktive Zwischenprodukte bilden, die eine Polymerisationsreaktion beispielsweise der oben genannten Monomeren und/oder Bindemittelharze auslösen können. Photoreaktionsstarter sind ebenfalls allgemein bekannt und können ebenfalls aus *[*Römpp Lexikon, Lacke und Druckfarben, Dr. Ulrich Zorll, Thieme Verlag Stuttgart-New York, 1998, S. 445/446*]* oder unter dem Stichwort 'Photoinitiatoren' entnommen werden.

Erfindungsgemäß besteht keine Einschränkung hinsichtlich der einzusetzenden photohärtbaren Monomeren oder Photoinitiatoren.

Die Erfindung betrifft bevorzugt Photolacke enthaltend
A) wenigstens ein "Pigment" im obigen Sinne, insbesondere in Mischung mit anderen Pigmenten oder eine darauf basierende erfindungsgemäße Pigmentpräparation,
B1) wenigstens ein photohärtbares Monomer,
B2) wenigstens einen Photoinitiator,
C1) gegebenenfalls ein organisches Lösungsmittel,
D) gegebenenfalls ein Dispergiermittel,
E) gegebenenfalls ein Bindemittelharz,
sowie gegebenenfalls weitere Zusätze.

Erfindungsgemäß besteht auch keine Einschränkung hinsichtlich der Technologie zur Erzeugung der gefärbten Bildelementmuster auf Basis der erfindungsgemäß zu verwendenden Pigmente oder festen Pigmentpräparationen. Neben dem oben beschriebenen photolithographischen Verfahren sind andere Verfahren wie Offset-Druck, chemisches Ätzen oder Ink Jet Druck ebenso geeignet. Die Auswahl der geeigneten Bindemittelharze und Lösungsmittel bzw. Pigment-Trägermedien sowie weitere Zusätze sind auf das jeweilige Verfahren abzustimmen. Beim Ink Jet Verfahren, worunter sowohl der thermische als auch mechanische und piezzo-mechanische Ink Jet Druck verstanden werden, kommen neben rein organischen auch wässrig-organische Trägermedien für die Pigmente und gegebenenfalls Bindemittelharze in Frage, wässrig-organische Trägermedien werden sogar bevorzugt, wobei als organische Lösungsmittel, die obengenannten in Frage kommen.

Die Erfindung betrifft auch Farbfilter, **dadurch gekennzeichnet, dass** sie wenigstens ein Fluor-substituiertes Perylen-Pigment enthalten, vorzugsweise ein Pigment der Formel (I), insbesondere ein Pigment der Formel (Ia). Ganz besonders bevorzugte Farbfilter enthalten wenigstens ein Pigment der Formel (II), (IV) oder (V). Im allgemeinen ist ein Farbfilter ein auf einem Träger ausgehärteter Photolack, wobei als Träger neben Glas auch andere Substrate wie Kunststoff-, Metall- oder Keramikplatten verstanden werden.

Die Erfindung betrifft auch Flüssigkristallanzeigen enthaltend wenigstens einen erfindungsgemäßen Farbfilter.

### Beispiele

### Beispiel 1

### a) Synthese

200g Phenol wurden bei 80°C vorgelegt. 0,1 mol Perylentetracarbonsäuredianhydrid (Persäure) und 0,3 mol Diethanolamin wurden zugegeben und der Ansatz in 30 min auf 120°C erhitzt. 0,225 mol p-Trifluormethoxyanilin wurden eingetragen und der Ansatz unter Abdestillieren von Phenol/Wasser für 5h auf 150°C erhitzt. Anschliessend wurde der Ansatz indirekt auf 90°C abgekühlt und 280 g Methanol zugetropft. Anschliessend wurde auf 40°C abgekühlt und isoliert. Das Produkt wurde mit 500g Methanol und dann mit 500 g Wasser gewaschen.

Der feuchte Presskuchen wurde in 800 ml 5%ige KOH-Lösung eingetragen, auf 80°C erwärmt und 1h nachgerührt, isoliert, mit Wasser neutral gewaschen und getrocknet. Das Produkt wurde in 1.500 g Schwefelsäure bei 20°C gelöst und auf 2.000 ml Methanol bei ca. 60 °C langsam ausgetragen. Die Mischung wurde mit Wasser verdünnt und das Produkt durch Filtration isoliert, gewaschen und getrocknet.

Ausbeute: 95% Perylen der Formel (II) 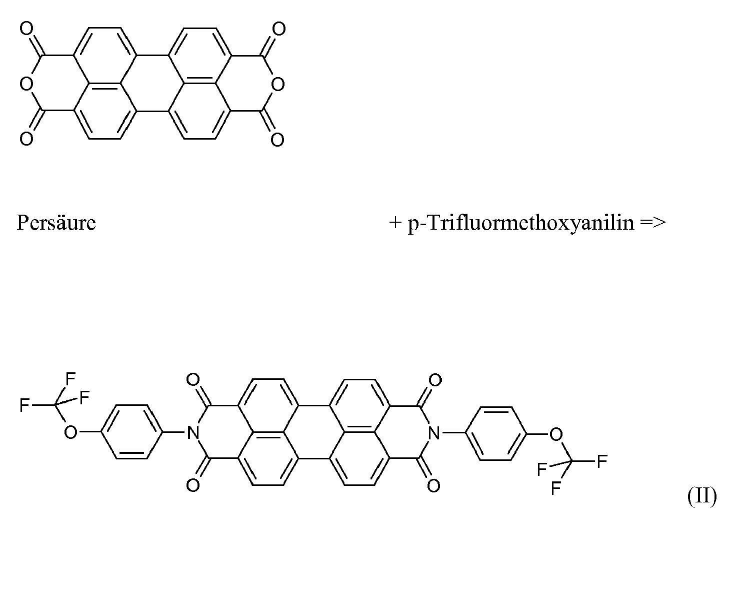 BET: 44m²/g

### b) Herstellung der PVC-Probe zur Bestimmung des Farbtons und der Dispergierhärte

Zur Bestimmung der Dispergierhärte wurden 100 g PVC-Paste , hergestellt aus 4,2 Teilen Vestolit® E 7004 (Emulsions-PVC-Pulver), 1,8 Teilen Diisooctylphthalat, 0,15 Teilen Stabilisator Baerostab® UBZ 770 (flüssiger Barium-Zink-Stabilisator) und 0,125 Teilen Moltopren® Weißpaste RUN 01 (Pigmentpräparation enthaltend 50% TiO₂), bei 150°C auf ein Labor-Mischwalzwerk der Fa. Collin aufgebracht. Der Walzenspalt betrug 0,8 mm.

0,1 g der Probe wurden auf das PVC-Fell aufgebracht und der Walzenpalt auf 0,12mm eingestellt. Das Walzenfell wurde abgenommen und wieder aufgegeben. Dieser Vorgang wurde achtmal wiederholt. Der Walzenspalt wurde auf 0,8 mm eingestellt und das Walzenfell abgenommen. Aus dem Walzenfell wurde ein Prüfmuster von 60 X 60 mm ausgestanzt.

Der Rest des Felles wurde nun auf das Walzwerk bei 25°C gegeben. Der Walzenspalt betrug 0,2 mm. Das Walzenfell wurde abgenommen und wieder aufgegeben. Dieser Vorgang wurde 15 mal wiederholt. Das vom Kaltwalzen nicht mehr glatte Fell wurde auf die Walze bei 150 °C und einem Walzenspalt von 0,8 mm gegeben. Nach 60 s wurde das Fell abgenommen und ein Prüfmuster von 60 X 60 mm ausgestanzt. Die Umdrehungszahl der Walze wurde immer konstant auf 20 upm und die Friktion auf 1:1,1 gehalten.

Die Dispergierhärte ist die prozentuale Zunahme der Farbstärke nach Walzen bei 25°C.
Dispergierhärte: 38

Die Herstellung der transparenten Prüfmuster erfolgte in gleicher Weise, allerdings unter der Verwendung einer Prüfpaste bestehend aus 4,2 Teilen Vestolit® E 7004 (Emulsions-PVC-Pulver), 1,8 Teilen Diisooctylphthalat, 0,15 Teilen Stabilisator Baerostab® UBZ 770 (flüssiger Barium-Zink-Stabilisator).

Die Farbtöne wurden mit einem Spektrometer der Fa. Gretag Macbeth bestimmt.

Dabei wurden die Proben mit 10° Beobachterwinkel, Lichtart D65 und ohne Glanzfalle gemessen.

Remissionsmessung:

| | | | | |
|---|---|---|---|---|
| L*: 62,18 | a*: 46,96 | b*: 4,63 | C*: 47,19 | h°: 5,63 |

Die Pigmente der Formel (III) bis (IX) wurden analog zum Beispiel 1 hergestellt.

### Verwendungsbeispiele

Herstellung einer roten Zubereitung und Verwendung zur Herstellung eines roten Farbfilters

**Verwendungsbeispiel 1 (erfindungsgemäß):**

| | |
|---|---|
| Einsatzpigment: | Pigment aus Beispiel 1 |

In einem Rührbehälter wurden 774 Gew.-Teile Methoxybutylacetat und 286 Gew.-Teile einer 21%igen Lösung eines alkalisch löslichen Copolymeren (Bindemittelharz) auf Basis von Benzylmethacrylat (70T) / 2-Hydroxyethylmethacrylat (15T) / Methacrylsäure (15T), Molgewicht ca. 25.000 g/mol, in Methoxypropylacetat homogen gemischt. Erhalten wurde eine "Zubereitung."

Anschließend wurden 100 Gew.Teile Pigment aus Beispiel 1, das zuvor bei 70°C auf eine Restfeuchte von weniger als 1 Gew% getrocknet wurde, homogen in die Zubereitung eingetragen.

Die Pigmentsuspension wurde in einer horizontalen, geschlossenen Perlmühle unter Einsatz von Yttrium-stabilisierten Zirkonoxid-Perlen (Durchmesser 0,6 bis 1,0 mm) gemahlen.

### Herstellung eines Photolacks

Zu 1000 Gew.-Teilen der so erhaltenen Zubereitung wurden 34,5 Gew.-Teile Trimethylolpropantriacrylat (monomerer Reaktivverdünner) und 13,8 Gew.Teile eines Photoreaktionsstarters auf Basis von Benzophenon und N,N'-Tetraethyl-4,4'-diaminobenzophenon im Verhältnis von 3 / 1 Gew.Teilen unter Rühren homogen eingetragen.

Man erhielt einen UV-strahlungshärtbaren Photolack, der auf einem transparenten Substrat aufgetragen und zum Farbfilter entwickelt wurde.

Hierzu wurde der Photolack mittels Spin-Coating auf ein 300 x 350 mm großes Stück gereinigtes Borosilikatglas (Corning® 7059, Owens Corning Corp.) beschichtet und bei 110°C 5 Minuten in einem Ofen unter Reinbedingungen zu einem ca. 1,5 - 2µm dicken Film getrocknet.

Anschließend wurde der Film nach Abkühlung mittels einer Negativmaske zur Erzielung des gewünschten Streifenbildmusters und einer Ultrahochdruck-Quecksilberdampflampe bei einer Dosis von 200 mJ / cm² UV-belichtet und dann mittels 0,06 %iger wässriger Kaliumhydroxidlösung bei Raumtemperatur entwickelt, mit vollentsalztem Wasser gereinigt und getrocknet. Anschließend erfolgte eine 30 minütige Nachhärtung bei 235°C in einem Ofen unter Reinbedingungen.

Der so erhaltene rote erfindungsgemäße Farbfilter 1 besaß gegenüber dem nicht-erfindungsgemäßen Farbfilter 2, hergestellt gemäß dem Verwendungsbeispiel 2 eine deutlich verbesserte spektrale Transparenz. Die Farbreinheit und Brillanz des Farbfilters 1 ist ausgezeichnet.

**Verwendungsbeispiel 2 (nicht erfindungsgemäß):**

| | |
|---|---|
| Einsatzpigment: | Pigment Rot 149 |

Analog dem Verwendungsbeispiel 1 wurden 100 Gew.Teile Pigment Rot 149, das zuvor bei 70°C auf eine Restfeuchte von weniger als 1 Gew% getrocknet wurde, homogen in die Zubereitung aus Verwendungsbeispiel 1 eingetragen.

Die erhaltene Pigmentsuspension wurde in einer horizontalen, geschlossenen Perlmühle unter Einsatz von Yttrium-stabilisierten Zirkonoxid-Perlen analog dem Verwendungsbeispiel 1 gemahlen.

Die Herstellung des Photolacks erfolgte ebenfalls analog zum Verwendungsbeispiel 1.

**Verwendungsbeispiel 3 (erfindungsgemäß):**

| | |
|---|---|
| Einsatzpigment: | 80% Pigment aus Beispiel 1 |
| | 20% mit Melamin interkalliertes Nickelazobarbitursäurepigment hergestellt nach Beispiel 2 aus DE 10 2005 033 581. |

Die Herstellung einer roten Zubereitung und Verwendung zur Herstellung eines roten Farbfilters erfolgte analog dem Verwendungsbeispiel 1.

Ein wie in Verwendungsbeispiel 1 beschrieben hergestellter Photolack und damit hergestellter roter erfindungsgemäßer Farbfilter besaß sehr gute spektrale Transparenzeigenschaften sowie ausgezeichnete Farbreinheit und Brillanz.

**Verwendungsbeispiel 4** (erfindungsgemäß):
Anlog Verwendungsbeispiel 1 wurde ein Farbfilter auf Basis der folgenden Pigmentmischung hergestellt.

| | |
|---|---|
| Einsatzpigment: | 40% Pigment aus Beispiel 1 |
| | 40% C.I. Pigment Rot 254 |
| | 20% mit Melamin interkalliertes Nickelazobarbitursäurepigment |

Der hergestellte Photolack und damit hergestellter roter erfindungsgemäßer Farbfilter besaß sehr gute spektrale Transparenzeigenschaften sowie ausgezeichnete Farbreinheit und Brillanz.

## Patentansprüche

1. Verwendung von Fluor-substituierten Perylenen in Farbfiltern für Flüssigkristallanzeigen.

2. Verwendung gemäß Anspruch 1, **dadurch gekennzeichnet, dass** das Fluor-substituierte Perylen der Formel (I) entspricht. 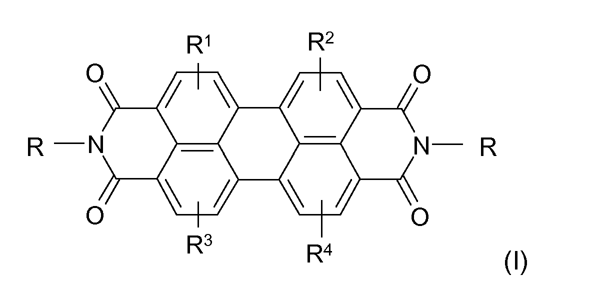 worin
R für H oder für einen ggf. substituierten organischen Rest steht und
R¹, R², R³ und R⁴ unabhängig voneinander für H, F, Cl, Br oder für einen ggf. substituierten organischen Rest stehen und dass wenigstens einer der Reste R, R¹, R², R³, R⁴ für Fluor steht oder wenigstens ein Fluoratom enthält.

3. Verwendung gemäß Anspruch 2, **dadurch gekennzeichnet, dass** der Rest R für einen durch ein oder mehrere Fluoratome substituierten Rest steht.

4. Verwendung gemäß Anspruch 1, **dadurch gekennzeichnet, dass** das Fluor-substituierte Perylen der Formel (Ia) entspricht 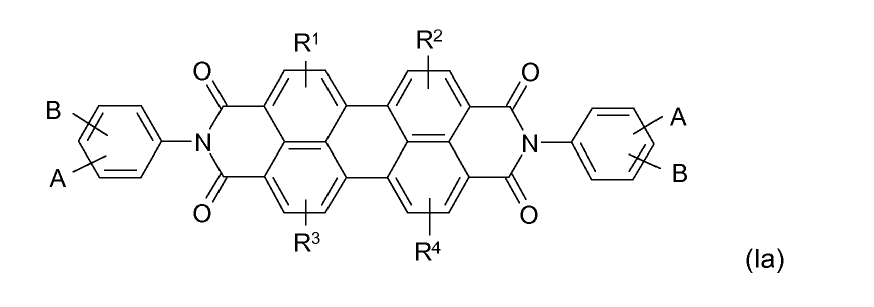 worin
A für Fluor oder einen durch ein oder mehrere Fluoratome substituierten organischen Rest steht,
B für H, F, Cl, Br oder für einen ggf. substituierten organischen Rest steht, der ggf. zusammen mit A einen Ring bilden kann, oder die Reste
A und B zusammen mit den C-Atomen des Phenylringes an denen sie gebunden sind einen annellierten Ring bilden und
R¹, R², R³ und R⁴ unabhängig voneinander für H, F, Cl, Br oder für einen ggf. substituierten organischen Rest stehen.

5. Verwendung gemäß Anspruch 4, **dadurch gekennzeichnet, dass** in Formel (Ia) R¹, R², R³, R⁴ vorzugsweise für Wasserstoff oder Fluor steht, der Rest B für H oder ggf. für Fluor-substituiertes C₁-C₈-Alkyl, insbesondere Methyl oder Trifluormethyl steht und
A für Fluor oder für C₁-C₈-Alkyl, insbesondere Methyl, C₁-C₈-Alkoxy, insbesondere Methoxy, Phenyl oder Phenoxy, das vorzugsweise jeweils durch ein oder mehrere Fluoratome substituiert ist, oder
A und B gemeinsam ein Brückenglied der Formel -O-CF₂-O-, -O-CF₂-CF₂-O-, -O-CHF-CHF-O- oder -O-CF₂-CF₂-CF₂-O-.bedeuten.

6. Verwendung gemäß Anspruch 1, **dadurch gekennzeichnet, dass** das Fluor-substituierte Perylen wenigstens einem Perylen der Formeln (II) bis (XII) entspricht,

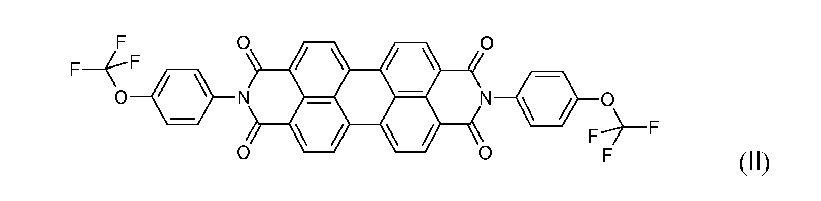

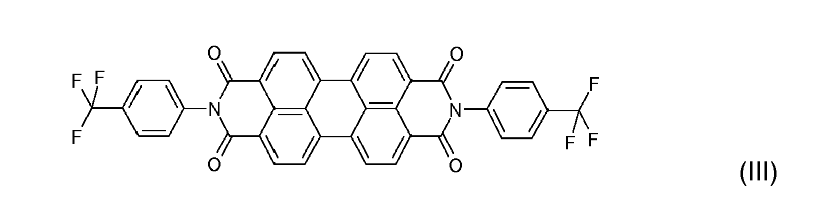

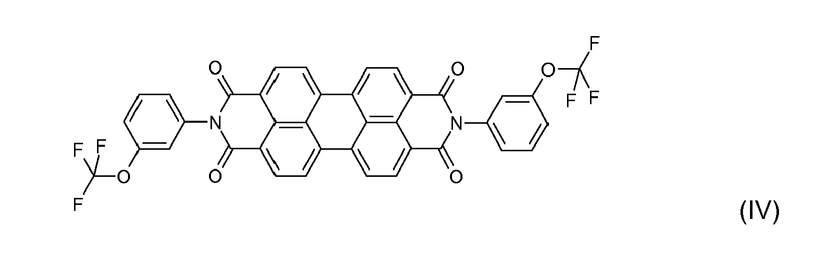

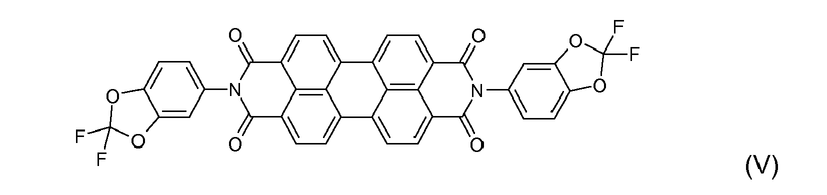

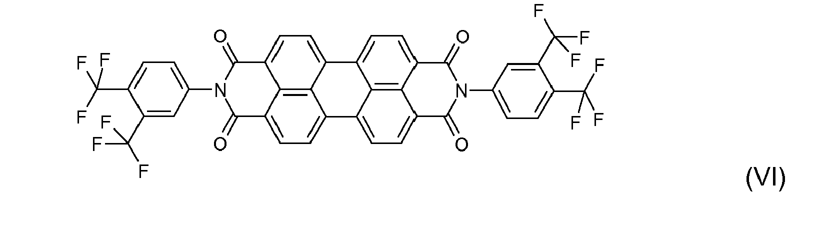

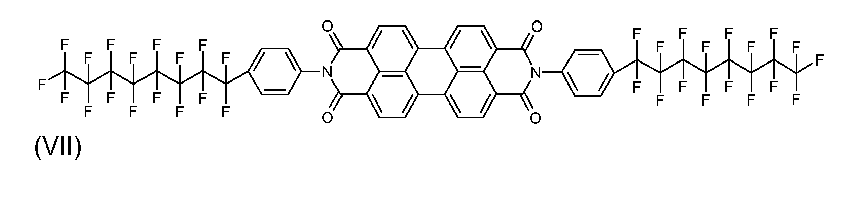

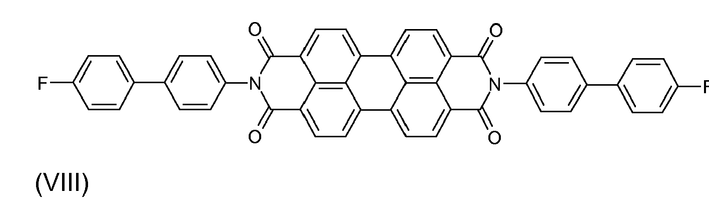

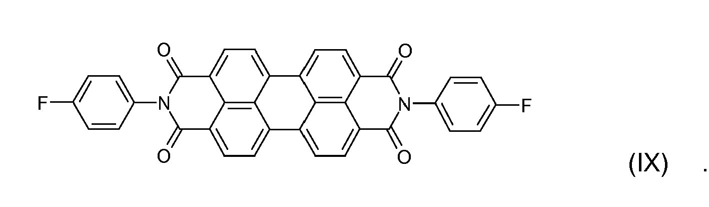

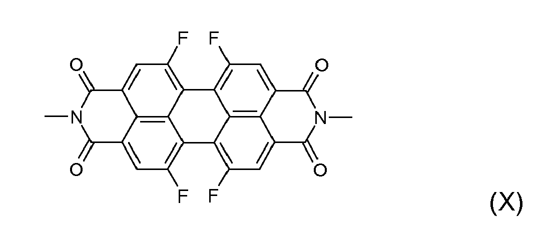

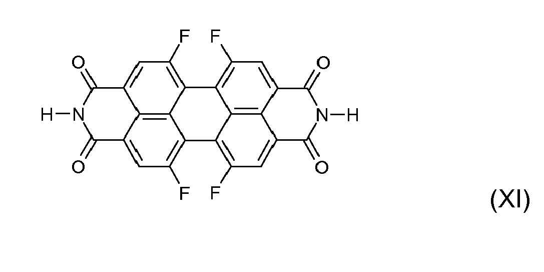

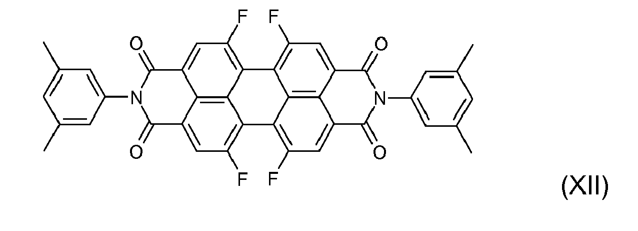

7. Verwendung nach Anspruch 1, **dadurch gekennzeichnet, dass** das Fluor-substituierte Perylen Pigment, insbesondere das der Formel (I) gemäß Anspruch 2 eine Oberfläche nach B.E.T. von 40 - 200 m²/g aufweist.

8. Verbindungen der Formel (II), (IV) oder (V), insbesondere Verbindungen der Formel (II)

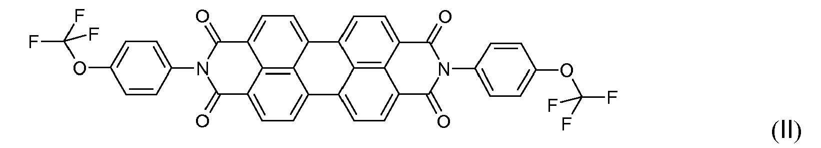

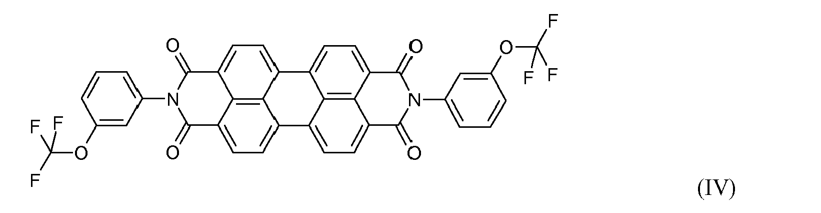

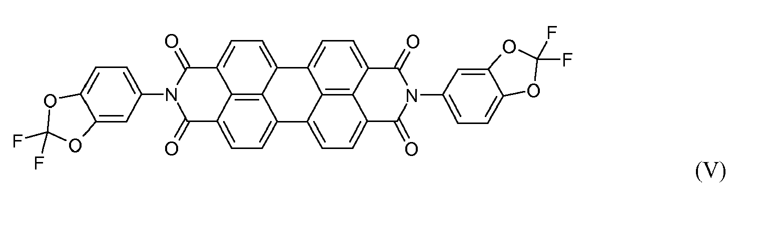

9. Photolack, enthaltend wenigstens ein photohärtbares Monomer, wenigstens einen Photoinitiator und wenigstens ein Fluor-substituiertes Perylen Pigment, insbesondere das der Formel (I) gemäß Anspruch 2.

10. Farbfilter, enthaltend wenigstens ein Fluor-substituiertes Perylen Pigment, insbesondere das der Formel (I) gemäß Anspruch 2.

11. Flüssigkristallanzeige, enthaltend wenigstens einen Farbfilter gemäß Anspruch 10.

12. Mischung, enthaltend neben wenigstens einem Fluor-substituierten Perylen, insbesondere wenigstens einem Pigment der Formel (I) gemäß Anspruch 2, wenigstens ein weiteres Rotpigment aus der Gruppe C.I. Pigment Rot 122, C.I. Pigment Rot 149, C.I. Pigment Rot 177, C.I. Pigment Rot 179, C.I. Pigment Rot 254 und C.I. Pigment Violett 19.

13. Mischung gemäß Anspruch 12, enthaltend zusätzlich ein oder mehrere Gelbpigmente oder Orangepigmente, insbesondere solche, die eine Absorptionsbande im Bereich von 400 bis 520 nm besitzen.

14. Mischung, enthaltend neben wenigstens einem Fluor-substituierten Perylen, insbesondere wenigstens einem Pigment der Formel (I) gemäß Anspruch 2 zusätzlich noch wenigstens ein Gelbpigment.

15. Verfahren zum Pigmentieren von Farbfiltern für LCD, **dadurch gekennzeichnet ist**, das der Farbfilter mit Fluor-substituierten Perylenen pigmentiert wird.
